# EUROPEAN PATENT SPECIFICATION

(11) **EP 1 151 755 B1**
(45) Date of publication and mention of the grant of the patent: **16.03.2005**
(21) Application number: 00201627.7
(22) Date of filing: 04.05.2000
(51) Int. Cl.: A61K 38/13, A61K 9/48, A61K 9/107, A61P 37/00, A61K 47/10, A61K 47/14, A61K 47/44

(54) **Pharmaceutical compositions comprising cyclosporin as active ingredient**
Pharmazeutische Zusammensetzung enthaltend Cyclosprin als aktiven Bestandteil
Compositions pharmaceutiques contenant la cyclosporine comme agent actif

(43) Date of publication of application: 07.11.2001
(73) Proprietor: Panacea Biotec Limited, 110 044 New Dehli (IN)
(72) Inventor: Singh, Amarjit, 110 044 New Delhi (IN); Jain, Rajesh, 110 044 New Delhi (IN)
(74) Representative: Wittop Koning, Tom Hugo

(56) References cited:
- EP-A- 0 982 035
- EP-A- 0 985 412
- GB-A- 2 228 198

## Description

### INTRODUCTION

The present invention relates to pharmaceutical compositions comprising Cyclosporin as active ingredient. The present invention also relates to novel alcohol free, free flowing, clear and transparent compositions comprising Cyclosporin as an active ingredient. The novel compositions are characterised in having increased bio-availability when the drug is formulated in a solubilised system and also amenable to convenient commercial production.

### BACKGROUND OF THE INVENTION

Cyclosporins comprise a class of structurally distinctive, cyclic, poly-N-methylated endecapeptides, commonly possessing pharmacological, in particular immunosuppressive, anti-inflammatory and/or anti-parasitic activity. The first of the Cyclosporins to be isolated was the naturally occurring fungal metabolite Ciclosporin or Cyclosporine, also known as Cyclosporin A and commercially available under several brands. Ciclosporin is the Cyclosporin of formula A. 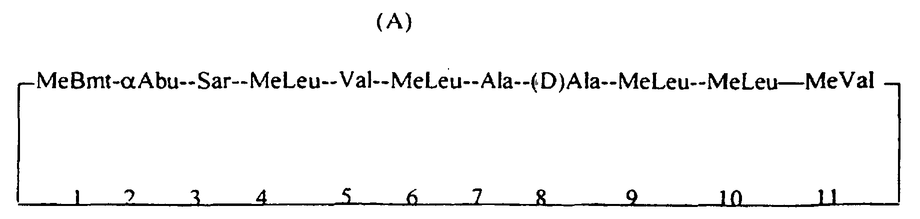 wherein - MeBmt- represents the N-Methyl-(4R)-4-but-2E-en-1-yl-4-methyl-(L) threonyl residue of formula B. 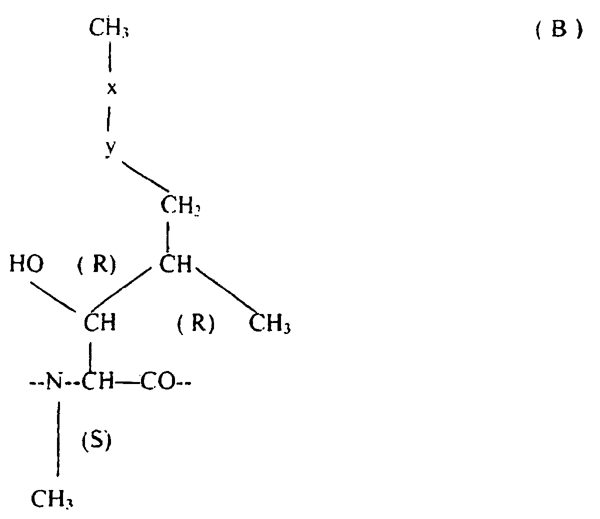 in which -x-y- is --CH =CH-- (trans).

Naturally occurring and semi-synthetic Cyclosporins, their classification, nomenclature etc. are known [c.f Traber et al. 1, Helv. Chim Acta. 60, 1247-1255 (1977): Traber et al. 2, Helv. Chim. Acta. ( 65 no. 162, 1655-1667 ( 1982)); Kobel et al., Europ. J. Applied Microbiology and Biotechnology 14, 273-240(1980); and von Wart -burg et al., Progress in allergy , 38,28-45(1986)]. US Patent Nos 4,108,985, 4,210,581 and 4,220,641; European Patent Publication Nos. 0034567 and 0056782: International Patent Publication no. WO 86/02080; Wenger 1, Transp. Proc. 15, Suppl. 1; 2230 (1983); Wenger 2, Angew. Chem. Int. Ed., 24,77 (1985); and Wenger 3, Progress in Chemistry of Organic natural Products 50, 123( 1986). Other Cyclosporins are known from U.S Patents 4,639,434; 4,703,033; 4,764,503, 4,885,276; 5,116,816; 5,122,511; 5,525,590; 5,643,870 and 5,767,069.

So far the primary area of clinical investigation for cyclosporins and in particular, Ciclosporin has been as an immunosuppressive agent, in particular in relation to its application to recipients of organ transplants, e.g. heart, lung, combined heart-lung, liver, kidney, pancreatic, bone-marrow, skin and corneal transplants and, in particular, allogenic organ transplants. In this field Cyclosporins, in particular ciclosporins have achieved a remarkable success. Among all the Cyclosporins, Cyclosporin A ( also known as Cyclosporine or Ciclosporin) has established its utility in the area of organ transplant and therapy of autoimmune diseases.

At the same time, applicability of Cyclosporins including Ciclosporin to various autoimmune diseases and to inflammatory conditions, in particular inflammatory conditions with an aetiology including an autoimmune component such as arthritis (for example rheumatoid arthritis, arthritis chronica progrediente and arthritis deformans) and rheumatic diseases, has been intensive and reports and results in vitro, in animal models and in clinical trials are wide-spread in the literature. Specific auto-immune diseases for which Cyclosporin and Ciclosporin therapy has been proposed or applied include, autoimmune hematological disorder (including e.g. hemolytic anaemia, aplastic anaemia, pure red cell anaemia and idiopathic thrombocytopaenia), systemic lupus erythematosus, poly-chondritis, sclerodoma, Wegener granulamatosis, dennatomyositis. chronic active hepatitis, myasthenia gravis, psoriasis, Steven-Johnson syndrome, idiopathic sprue, autoimmune inflammatory bowel disease (including e.g. ulcerative colitis and Crohn's disease) endocrine opthalmopathy, Graves disease, sarcoidosis, multiple sclerosis, primary biliary cirrhosis, juvenile diabetes (diabetes mellitus type I), uveitis (anterior and posterior), keratoconjunctivitis sicca and vernal keratoconjunctivitis, interstitial lung fibrosis, psoriatic arthirits and glomerulonephritis (with and without nephrotic syndrome, e.g. including idiopathic nephrotic syndrome or minimal change nephropathy).

Further areas of investigation for cyclosporins include potential applicability as an anti-parasitic, in particular anti-protozoal agent, with possible uses suggested including treatment of malaria, coccidiomycosis and schistosomiasis and, yet more recently, use as an agent for reversing or abrogating anti-neoplastic agent resistance in tumours and the like.

Although Cyclosporin A is the most widely used amongst all the immunosuppresants available so far, it suffers from a serious drawback of poor bio-availability. Cyclosporin blood levels have to be maintained within a specified range to achieve the effective therapy. The required range varies according to the clinical status of the patient.

Because of poor and variable bioavailability daily dosages needed to achieve the desired blood levels need to be varied considerably in the existing dosage forms of Cyclosporin and a concomitant monitoring of blood levels is essential. This adds an additional cost to the therapy.

In order to improve the bio-availibility several attempts have been made to improve formulations of Cyclosporin.
Han Gua Patent (Chinese Patent CN 1.121.694) explains the active compound of Cyclosporin, fatty acid sugar ester and diluent carrier having good bio-availability. However, this compound suffers from the drawback that diluent degrades due to hygroscopicity of sugar ester and the stability is not of desired standards, (See also Pharmaceutical Research, Volume 6, No. 11, 1989, P958, "Solid Surfactant Solution of active Ingredients in Sugar Ester" and International Journal of Pharmaceutics, Vol. 92, 1993, P197," Application of sucrose laurate a new pharmaceutical excipient, in Peroral formulation of Cyclosporin A").

Chinese Patent CN 1.121 694 having equivalent EP 0702562 describes a powder dosage form of cyclosporin possessing comparatively higher stability and to some extent bio-availability when compared to the earlier formulations. This art describes adsorption of Cyclosporin A with appropriate solvents onto an adsorbent along with a nonionic hydrophillic surfactant. The final product does not contain the solvent as this evaporates during the process of manufacturing. Thus this product does not suffer from the disadvantage arising out of solvent evaporation during shelf life and hence stability problems. The various pharmaceutical surfactants, polyhydric alcohols and solvents are well known to the art. The adsorbent used is Colloidal Silicon Dioxide. The blood level arising out of such product have been compared with the standard formulations as per US patent no. 4, 388, 307 with significant improvement in bioavailability. However, when compared with the micro-emulsion based formulations (described later) these formulations do not show any advantage as the drug is adsorbed on solid surface and needs an additional process of dissolution prior to become bioavailable.

The effect of sucrose laurate on the gastrointestinal absorption of Cyclosporine is also described (Lerk-PC; Sucker-H, International Journal of Pharmaceutics; 1993; 92; (May 3); 197-202). The evaluation of the dosage form containing sucrose Laurate was found to enhance the in vitro absorption of Cyclosporine when normal epithelial tissue and Peyer's patch tissue of guinea pigs were used. Compared to the commercially available drinking solution, absorption was raised by a factor of 10. Excess amount of surfactant reduced drug absorption. Despite large excess of Sucrose laurate, the absorption of Cyclosporin was still superior to the drinking solution. Choleic acid was also found to increase absorption by a factor of 5-6. A comparison of the absorption between normal epithelial and Peyer's patch tissues indicated that the absorption by endocytosis does not contribute significantly to the overall absorption of Cyclosporin. It was concluded that preliminary formulation experiments showed that a solid oral dosage form of Cyclosporin could be made using sucrose laurate as an excipient.

Abdallah-HY; Mayersohn-M. Pharmaceutical Research; 1991;8(Apr);518-522 reported several formulations of Cyclosporin were prepared and examined in vitro and in dogs.A tablet formulation was then selected for comparison with the commercial oil solution placed in a soft gelatin capsule in a randomized crossover study in dogs. Compared with an intravenous dose of the drug, absolute bioavailability was 46+11.1 and 45+ 9.9% for the capsules and tablets, respectively. Maximum concentration, time to reach maximum concentration and mean absorption time were not significantly different between the 2 formulations. It was concluded that the tablet formulation of Cyclosporin is equivalent in dogs to the commercial dosage form packed into soft gelatin capsules.

US patent no. 505 1402 describes that Cyclosporin may be rendered more soluble by the concomitant administration of α-Cyclodextrin, either separately, but essentially simultaneously or, preferably, in admixture.

US Patent No. 4990337 describes a formulation comprising a Cyclosporin in admixture with at least one mono or diglyceride of a C₆- C₁₀ fatty acid sufficient to dissolve the Cyclosporin. The resulting solution can then easily be emulsified in water or an aqueous fluid.

Freeze dried liposome mixture containing Cyclosporin has been described in US Patent no. 4963362. This invention provides a freeze-dried potential liposome mixture having an amphipathic lipid and a Cyclosporin or derivative thereof for use in possible liposome delivery of Cyclosporin into cells. A method to produce the freeze-dried mixture is also disclosed. When reconstituted to yield liposomes in an aqueous medium, substantially all of the Cyclosporin present in the freeze-dried mixture is encapsulated in the liposomes.

Other galenic improvements in Cyclosporin emulsion formulations recorded in prior art are the use of tocopherol derivatives (EP 0724452), tocopheryl polyethylene glycol carboxylic acid ester (EP 0712631), dimethylisosorbide (EP 0711550, EP 0650721), alkylene polyether or polyester (WO 9423733), emulsion compositions (EP 0694308), anhydromannitol oleylether, lactoglyceride, citroglycerides (EP 656212), phosphatidyl ethanolamine (EP 0651995), as surfactants and stabilizers etc.

Three Patent Applications namely European Patent App. EP-A-0650721, EP-A-0.711.550 and WO 96/13273 describe the use of Dimethylisosorbide as a co-surfactant or a hydrophillic phase along with other ingredients to enhance the absorption of Cyclosporin.

Thomas Cavanak in U.S. Pat. no. 4,388,307 have described compositions employing ethanol, olive oil as carrier medium in conjunction with Labrafil as surfactant. These compositions yield crude emulsions on dilution with water. The bio-availability levels using these dosage forms are low and exhibit wide inter-and intra-individual variations. Such dosage forms provide an average absolute bioavailability of ca 30%. Reported variation in bio-availability between subjects varies between a few percent for some patients to as much as 90% or more for others. Also a marked change in bio-availability for individuals with time is frequently observed.

U.S. Patent no. 5,977,066 discloses oral compositions using monoglycerides, diglycerides and triglycerides alongwith castor oil derivatives. Compositions of the present invention do not contain fatty materials like mono-,di-, or triglycerides.
U.S. Patent no. 6,022,852 discloses the use of tocopherol polyethylene glycol 1000 succinate for formulating Cyclosporin A.

Hong et al. In U.S. Patent no. 6,028,067 disclose the use of lipophilic solvent chosen from an alkyl ester of polycarboxylic acid and a carboxylic acid ester of polyols, alongwith an oil; and a surfactant to form microemulsion preconcentrate of cyclosporin A. Such lipophilic solvents are not present in the present invention.

Sherman Bernard Charles (WO9848779) claim an emulsion preconcentrate comprising a cyclosporin dissolved in a solvent system comprising acetylated monoglycerides, and a surfactant.

Robert Floc'h et al. In U.S. Patent no. 5, 827, 822 disclosed aqueous suspension of amorphous Cyclosporin nanoparticles wherein at least 50 percent of the drug is present as particles of less than about 1 µm.

One of the most significant attempt to improve bio-availability of Cyclosporin from its dosage form is the described in US patent no. 5. 342, 625. This art describes use of microemulsion pre-concentrate consisting of a three phase systems i.e. (1) a hydrophilic phase component (2) a lipophilic phase component and (3) a surfactant. Such composition has alcohol as an essential ingredient. Such composition upon dilution with water provides an oil-in-water microemulsion with an average particle size of less than 1000A°. Such an enhanced surface area results in increased bio-availability of Cyclosporin when compared with conventional dosage forms. A comparison of bio-availability from micro-emulsion dosage form (Composition I from US patent no. 5, 342, 625) with the conventional ethanol/oil based dosage form earlier reported in US patent no. 4, 388, 307 has been performed in healthy human volunteers and reported in US patent no. 5,342, 625. Bio-availability level of 149.0% (± 48) is recorded for composition I as compared to composition X (for which bio-availability achieved is set as 100%). The mean AUC levels from composition I were 40% higher when compared to those from composition X but still had a high variation of 20%.

Some of the later U.S. Patents 5866159, 5916589, 5962014, 5962017, 6007840 and 6024978 also pertain to oil-in-water microemulsion compositions having particle size less than 2000Å.
Although the compositions which yield oil-in-water microemulsions show much better bioavailability and reduced variability, the requirement to convert drug into micro emulsion form utilizes very high concentrations of surfactants like polyoxyethylene hydrogenated castor oil. Such surfactants are known to cause toxicity problems like nephrotoxicity, fat embolism and anaphylactic reactions (Ref. : Handbook of Pharmaceutical Excipients, Second Edition, Eds. Ainley Wade and P.J. Weller, The Pharmaceutical Press, 1994, p. 371 - 374). Moreover high amounts of surfactants unnecessarily increases the cost of the product.

The compositions of the European Patent Application no. 0985412 which describes micellar solubilization of Cyclosporin also suffer from similar disadvantages.

Alcohol is an essential part of most of the marketed compositions as is evident from the products available in the market (Sandimun [US Pat. No. 4, 388, 307] and Neoral [US Pat. No. 5,342, 625]) both of which contain Alcohol. Such compositions suffer from severe drawback of instability due to evaporation of a low boiling solvent like Alcohol. This is particularly true as the products are used in home environment, which cannot be precisely controlled with respect to temperature. Although very expensive cumbersome technology (such as cold formed Aluminium/ Aluminium Blister packs) is adopted to protect these products, yet the problem of instability is not completely solved. The stability problems are evident from strict storage conditions and usage requirements as declared either on the labels or package inserts of commercial products Sandimun, and Neoral drink solutions and capsules. Some of the examples are:
1. There is a requirement of storage of product below 30°C at the same time refrigeration is prohibited. This means that a patient using this product in a tropical country need to have an air-conditioned home environment. This is not only a limiting factor in use of this product but sometimes in economically backward countries it may not be possible that every person using the product has an air-conditioned storage area. Sometimes factors like prolong electricity failure and mechanical and electrical defects in air-conditioning system can cause instability problems to these products rendering them unstable for use.
2. There is also an a statement in Packing insert of Sandimun and Neoral drink solutions that " Sandimun Neoral solution should be used within 2 months of opening the bottle and be stored between 15° and 30°C, preferably not below 20°C for prolonged periods, as it contains oily components of natural origin which tend to solidify at low temperatures. A jelly-like formation may occur below 20°C, which is however reversible at temperatures up to 30°C. Minor flakes or a slight sediment may still be observed. These phenomena do not affect the efficacy and safety of the product, and the dosing by means of the pipette remains accurate. " indicating instability problems.

US Patent no. 5, 639, 724 discloses pharmaceutical compositions comprising Cyclosporin, transesterification product of a natural vegetable oil with glycerol which is exemplified in the specification as MAISINE (transesterification product of corn oil and glycerol) which is an essential component of the compositions. The cyclosporin must be mixed with a transesterfication product of a natural vegetable oil with glycerol. These compositions are not useful as drink solutions because of formation of jelly like lumps, since the transesterfication product is a jelly like substance at room temprature. Such composition also preferably require the use of alcohol. This compositions compares its bioavailability with that of older and inferior compositions based on US patent no. 4, 388, 307 and does not compare bioavailability with a more recently marketed compositions (NEORAL) as defined in US patent no. 5, 342, 625. US Patent No. 5,639,724 discloses the use of Labrafil as a preferred ingredient to be added to the composition of Cyclosporin and MAISINE for a drink solution. However, this patent does not address the problem of flake like substances formed by the presence of MAISINE even though Labrafil has been added to the composition.

The major consideration here is the accurate measurement of dose in Cyclosporin which is an essential feature because of the narrow therapeutic condition of the drug i.e. below threshold the organ rejection occurs and above a particular level the drug causes severe toxic reactions. The presence of jelly like substances or flakes make accurate dose measurement difficult.

Our attempts to formulate Cyclosporin containing significantly reduced amounts of surfactant like polyoxyethylene hydrogenated castor oil have surprisingly resulted in compositions which on dilution with water yield oil-in-water emulsion having very narrow globule size distribution and have an average globule size ranging between 200 - 600 nm (determined using Photon Correlation Spectroscopy). Further such compositions are also devoid of other drawbacks associated with compositions of prior art.

Composition of the present invention do not contain volatile solvents like alcohol.

The compositions are stable between wide range of temperature i.e. 15° - 45°C and do not congeal or flake at lower temperatures.

The drug is present in solubilised form after dilution and does not precipitate out.

The compositions are hydrophilic and can take upto 10% w/w of water without drug crystallizing out of solution. This is advantageous in soft gelatin capsules where migration of water from shell can cause precipitation of drug.

The compositions can be formulated as a drink solution or suitably encapsulated.
The compositions do not require specialized and costly packaging.
Further compositions of the present invention are also devoid of "lipophilic component" as described in the US Patents 5,342,625 and 5,741,512. This lipophilic component has been defined in the said patents as solvents which are devoid or substantially devoid of surfactant function and non-miscible with the selected hydrophilic phase. The compositions of the present invention are substantially devoid of such "lipophilic components" and hence also devoid of the defects associated with such fatty materials.

Compositions according to this invention may be formulated for oral administration including but not limited to drink solutions or formulated as hard or soft gelatin capsules.The capsules may be gelatin or cellulose capsules or two piece hard shell capsules. Drink solution formulations may be diluted with water or aqueous medium and the lipophilic Cyclosporin drug is maintained in a solubilized state, hence making the drug bioavailable in therapeutic concentrations.

Oral solution concentrates are to be diluted prior to intake and are used as start up therapies. These dosage forms provide more flexibility in dosage adjustments to achieve the optimum therapeutic concentrations as desired by the physicians. The second type of dosage forms are unit dosage forms for example capsules, generally soft or hard gelatin capsules or cellulose capsules or two piece hard shell capsules.

### SUMMARY OF THE INVENTION

In accordance with the present invention there is described a homogenous substantially alcohol free, composition of Cyclosporin which comprises a Cyclosporin in a carrier medium comprising propylene glycol, esters of propylene glycol with C4 to C12 fatty acids and polyoxyethylene hydrogenated castor oils wherein the ingredients are present in the following range :

| | |
|---|---|
| Cyclosporin A | 1 - 25 %w/w |
| Propylene Glycol | 2 - 70% w/w |
| Esters of propylene glycol with C4 to C12 fatty acids | 15 - 60% w/w |
| Polyoxyethylene hydrogenated Castor oils | 5 - 20% w/w |

Wherein such compositions on dilution yield oil-in-water emulsions containing drug dissolved in the globules. The globules have average size within a narrow range of 200 - 600 nm.

### DETAILED DESCRIPTION OF THE INVENTION

In accordance with the present invention there is described a homogenous substantially alcohol free, composition of Cyclosporin which comprises a Cyclosporin in a carrier medium comprising propylene glycol, esters of propylene glycol with C4 to C12 fatty acids and polyoxyethylene hydrogenated castor oils wherein the ingredients are present in the following range :

| | |
|---|---|
| Cyclosporin A | 1 - 25 %w/w |
| Propylene Glycol | 2 - 70% w/w |
| Esters of propylene glycol with C4 to C12 fatty acids | 15 - 60% w/w |
| Polyoxyethylene hydrogenated Castor oils | 5 - 20% w/w |

Wherein such compositions on dilution yield oil-in-water emulsions containing drug dissolved in the emulsion droplets or globules. The globules have average size within a narrow range of 200 - 600 nm.

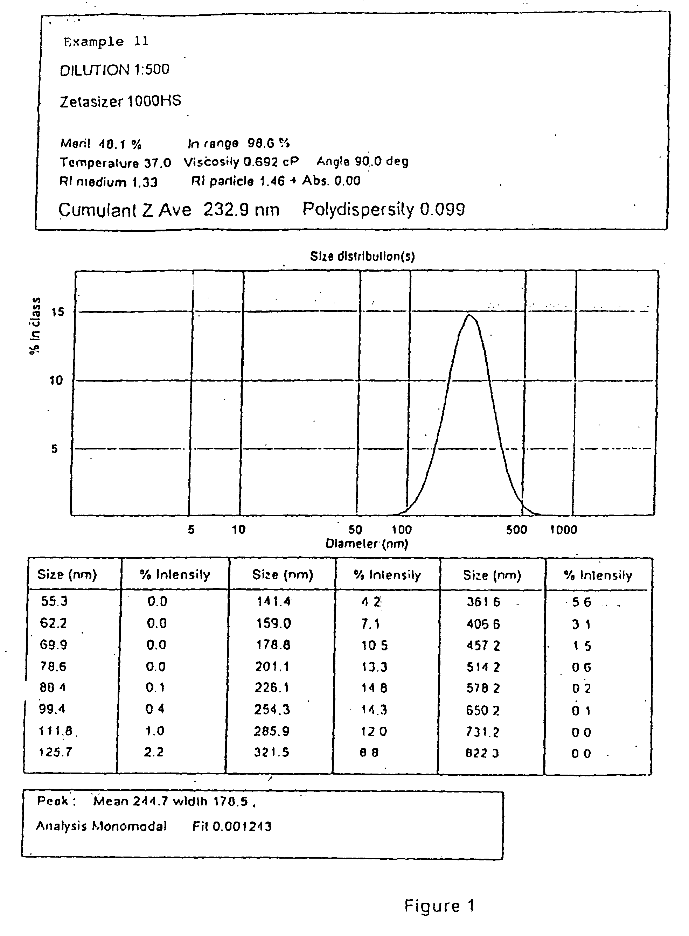

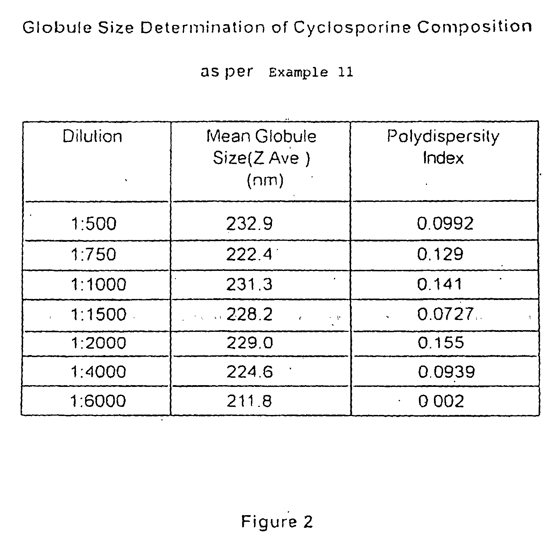

The size distribution of one such composition (as per example 11) is shown in Fig. 1 which shows an average globule size of approximately 225 nm. The average globule size determined at different dilution levels is shown in Fig 2.

In another preferred embodiment of the invention the composition further comprises Triacetin or Glycerol triacetate. The glycerol triacetate may be present in the range of 0% to 10% w/w.

In another embodiment of the present invention the composition further comprises Oleic Acid in the range of 0 to 60% w/w. Oleic Acid may partially or completely replace the esters of propylene glycol with C4 to C12 fatty acids.
In another embodiment of the present invention the composition further comprises Antioxidants in the range of 0 to 5% w/w. The antioxidants can be selected from Butylated Hydroxy Anisole,Bytulated Hydroxy Toluene, Tocopherylacetate, d-α-tocopheryl polyethylene glycol 1000 succinate or a mixture thereof. Other antioxidants may also be used. The use of such compounds is only due to their antioxidant activity and they may not contribute as solvents for Cyclosporin in the concentrations used.

In preferred embodiment of the invention is a composition comprising :

| | |
|---|---|
| Cyclosporin | 5 - 15 %w/w |
| Propylene Glycol | 5 - 50% w/w |
| Esters of propylene glycol with C4 to C12 fatty acids | 25 - 45% w/w |
| Polyoxyethylene hydrogenated Castor oils | 10 - 20% w/w |

In another embodiment of the invention the compositions are clear, stable, transparent, easily flowable and easily measurable at a wide range of temperature of 15° to 45°C.

The amount of all of the ingredients of the composition disclosed above equals to 100%.

The systems of the present invention are single phase systems. The expression "single phase" should be implied to mean a phase wherein the drug is solubilized using suitable blend of surfactant(s)/ co-surfactant(s).

The cyclosporins used in the compositions may be selected from cyclosporin A, cyclosporin D, cyclosporin G or any other known cyclosporin. It is preferred that cyclosporin A be used.

The composition can be incoporated into the capsule shells by conventional procedures as described in standard texts. ("The theory and practice of Industrial Pharmacy"by Leon Lachman et al. Third edition, LEA AND FEBIGER, USA).

The Soft Gelatin and two piece hard shell Capsules have a very distinct advantage of ease of carrying and administration as compared with oral solutions. These dosage forms hence contribute to a very large segment of commercial market. To aid the filling of the composition into two piece hard shell capsules suitable viscosity imparting agents known in the art, may be added. These may be selected from natural gums like Xanthan gum, Karaya gum, Guar gum, Acacia and the like or semi-synthetic or synthetic polymers like cellulosics e.g. hydrxypropyl cellulose. Hydroxypropylmethyl cellulose, Carboxy methyl cellulose; Acrylates like polymethyl methacrylic acid; carbomers like Carbopol 934, Carbopol 940 (BF Goodrich, USA); silicon dioxide.

Such compositions of the present invention which are solubilized systems and substantially free of C₁₋₅ alkanols such as ethanol are distinctly advantageous over the ones described in US patent no. 5, 342, 625 with respect to manufacturing and distribution in the tropical countries.

It is most beneficial in context of hot tropical countries where loss of C₁₋₅ alkanols such as ethanols are more due to evaporation.

Preferably the esters of propylene glycol with C4 to C12 fatty acids include mono-, di- or mono- and di- esters of propylene glycol with C4 to C12 fatty acids. Suitable products include propylene glycol laurate, propylene glycol dicaprylate/dicaprate, propylene glycol dioctanoate, propylene glycol monolaurate and the like. Especially suitable are the products commercially available under the trade names Lauroglycol, Neobee M-20, Capryol and Miglyol - 840.

Preferably the Polyoxyethylene hydrogenated castor oil is Polyoxyethylene 40 Hydrogenated Castor oil. Especially suitable is product available under the trade name Cremophor RH 40.

The term "easily measurable" has been used due to the characteristic features of the drug Cyclosporin. Cyclosporin requires accurate dose measurement because of its narrow therapeutic index. Most of the drink solution packs are provided with a pipette or a syringe for accurate dose measurement. This warrants that the solution is a sufficiently thin liquid to permit ease of measurement and not a semi solid mass and also it should be devoid of any flakes, jelly like formations or other sediments which can cause non-homogeneity in the dose. The composition of our invention possesses all the desired characteristics and hence is easily measurable as far as the dose requirements is concerned.

Compositions according to this invention may be formulated as drink solutions or diluted as a drink solution or formulated as soft gelatin capsules or as two piece hard shell capsules.

Several compositions as per this invention with different ranges of ingredients were subjected to commercial production trials and shelf-life stability studies and the inventors were successful in arriving at a composition which was easy to manufacture and stable for long periods of time when tested by accelerated stability studies.

Moreover when tested on healthy human volunteers, the composition(s) of this invention was found to have excellent bio-availability of Cyclosporin and were also found to be bioequivalent with commercial product. The comparative results are collected in Table I.

**Table 1. :**

| **Plasma concentration of Cyclosporine (ng/ml) following administration of a single oral dose of 2.5 mg/kg body weight. (Mean ± S.D.)** | | | |
|---|---|---|---|
| **Product** | **Cmax** | **Tmax** | **AUC** |
| Neoral Mfd. By : Sandoz | 723.21 ± 278.57 | 2.20 ± 0.40 | 3203.6 ± 1364.0 |
| Composition as per Example 12. | 668.32 ± 233.19 | 2.61 ± 0.38 | 3048.71 ± 1180.23 |

The invention will now be described with reference to the accompanying examples which should not be construed to limit the scope of the invention :

### EXAMPLE 1 (PRIOR ART)

| | **COMPONENT** | **AMOUNT** |
|---|---|---|
| a) | Cyclosporin | 100 mg (= ca. 10.5%) |
| b) | Maisine | 550 mg (= ca. 57.8%) |
| c) | Labrafil M 2125 | 300 mg (= ca. 33.5%) |
| | TOTAL | 950 mg |

The mixture obtained was a semi-solid mass at room temperature suitable only for soft gelatin capsule formulation.

### EXAMPLE 2 (PRIOR ART)

| | **COMPONENT** | **AMOUNT** |
|---|---|---|
| a) | Cyclosporin | 100 mg (= ca. 10.5%) |
| b) | Maisine | 490 mg (= ca. 52%) |
| c) | Labrafil M 2125 | 300 mg (= ca. 31.5%) |
| d) | Cremophore RH40 | 60 mg (= ca. 6.3%) |
| | TOTAL | 950 mg |

The mixture obtained was a semi-solid mass at room temperature suitable only for soft gelatin capsule formulation.

### EXAMPLE 3 (PRIOR ART)

| | **COMPONENT** | **AMOUNT** |
|---|---|---|
| a) | Cyclosporin | 100 mg (= ca. 10.5%) |
| b) | Maisine | 850 mg (= ca. 52%) |
| | TOTAL | 950 mg |

The mixture obtained was a semi-solid mass at room temperature suitable only for soft gelatin capsule formulation.

### EXAMPLE 4 (PRIOR ART)

| | **COMPONENT** | **AMOUNT** |
|---|---|---|
| a) | Cyclosporin | 100 mg |
| b) | Propylene Glycol | 200 mg |
| c) | Cremophore RH40 | 350 mg |
| d) | Labrafil M1944 | 200 mg |
| e) | Maisine | 150 mg |
| | TOTAL | 1000 mg |

The composition obtained was a clear, homogenous liquid at a temperatures between 25° to 30°C, but at temperatures below 20°C jelly like flakes separated out.
Examples 5 to 15 relate to cyclosporin compositions of the present invention which can be used as a drink solution or can be suitably encapsulated.

### Example 5

Composition for two piece hard shell capsule

| | |
|---|---|
| Cyclosporin | 10 g |
| Propylene Glycol | 35 g |
| Cremophor RH 40 | 15 g |
| Propylene glycol laurate | 40 g |

Propylene Glycol was mixed with Cremophor RH 40 and heated upto 55 to 60°C and Cyclosporin was dissolved in the resultant. Propylene glycol laurate was then added to the bulk mixture and mixed. The resultant mixture was then filtered. The above composition may be filled in two piece hard shell capsules made up of materials like gelatin or cellulosics (e.g. Hydroxy propyl methyl cellulose based capsules like LICAPS ™) using suitably modified machines for filling liquids in two piece hard capsules. The capsules may be sealed using Qualiseal technology. The capsule shell may further comprise plasticizers, colorants, release modifiers, and the like.

### Example 6

| | |
|---|---|
| Cyclosporin | 10 g |
| Propylene Glycol | 40 g |
| Cremophor RH 40 | 14 g |
| Propylene glycol dicaprylate/dicaprate | 36 g |

Propylene Glycol was mixed with Cremophor RH 40 and heated upto 55 to 60°C and Cyclosporin was dissolved in the resultant. Propylene glycol dicaprylate/dicaprate was then added to the bulk mixture and mixed. The resultant mixture was then filtered.

### Example 7

| | |
|---|---|
| Cyclosporin | 10 g |
| Propylene Glycol | 40 g |
| Cremophor RH 40 | 15 g |
| Propylene glycol dioctanoate | 35 g |

Propylene Glycol was mixed with Cremophor RH 40 and heated upto 55 to 60°C and Cyclosporin was dissolved in the resultant. Propylene glycol dioctanoate was then added to the bulk mixture and mixed. The resultant mixture was then filtered.

### Example 8

| | |
|---|---|
| Cyclosporin | 10 g |
| Propylene Glycol | 40 g |
| Cremophor RH 40 | 10 g |
| Propylene glycol laurate | 30 g |
| Propylene glycol dicaprylate/dicaprate | 7 g |
| Triacetin | 3 g |

Propylene Glycol was mixed with Cremophor RH 40 and heated upto 55 to 60°C and Cyclosporin was dissolved in the resultant. Propylene glycol laurate was then added to the bulk mixture and mixed. To this was added propylene glycol dicaprylate/dicaprate followed by Triacetin. The resultant mixture was then filtered.

### Example 9

| | |
|---|---|
| Cyclosporin | 10 g |
| Propylene Glycol | 40 g |
| Cremophor RH 40 | 10 g |
| Propylene glycol monolaurate | 40 g |

Propylene Glycol was mixed with Cremophor RH 40 and heated upto 55 to 60°C and Cyclosporin was dissolved in the resultant. Propylene glycol monolaurate was then added to the bulk mixture and mixed. The resultant mixture was then filtered.

### Example 10 (excluded from the invention)

| | |
|---|---|
| Cyclosporin | 10 g |
| Propylene Glycol | 5 g |
| Cremophor RH 40 | 25 g |
| Propylene Glycol monolaurate | 30 g |
| Oleic Acid | 27 g |
| Triacetin | 2.93 g |
| Tocopheryl acetate | 0.07 g |

Propylene Glycol was mixed with Cremophor RH 40 and heated upto 55 to 60°C and Cyclosporin was dissolved in the resultant. Propylene glycol monolaurate was then added to the bulk mixture and mixed. To this was added Oleic acid, Triacetin and Tocopherylacetate. The resultant mixture was then filtered.

### Example 11

| | |
|---|---|
| Cyclosporin | 10 g |
| Propylene Glycol | 39.8 g |
| Cremophor RH 40 | 15 g |
| Propylene Glycol monolaurate | 35 g |
| Tocopheryl acetate | 0.2 g |

Propylene Glycol was mixed with Cremophor RH 40 and heated upto 55 to 60°C and Cyclosporin was dissolved in the resultant. Propylene glycol monolaurate was then added to the bulk mixture followed by Tocopheryl acetate and mixed. The resultant mixture was then filtered. This composition when diluted with water yields dispersions having average globule size of approximately 225 nm. The globule size distribution curve is shown in Fig. 1 and the average globule size at different dilution levels is shown in Fig. 2.

### Example 12

| | |
|---|---|
| Cyclosporin | 10 g |
| Propylene Glycol | 10 g |
| Cremophor RH 40 | 10 g |
| Oleic Acid | 30 g |
| Propylene glycol monolaurate | 38 g |
| Vitamin E TPGS | 2 g |

Propylene Glycol was mixed with Cremophor RH 40 and heated upto 55 to 60°C and Cyclosporin was dissolved in the resultant. Oleic Acid was then added to the bulk mixture and mixed. To this was added propylene glycol monolaurate and Vitamin E TPGS and mixed. The resultant mixture was then filtered.

### Example 13

| | |
|---|---|
| Cyclosporin | 10 g |
| Propylene Glycol | 67 g |
| Cremophor RH 40 | 5.0 g |
| Propylene Glycol monolaurate | 16 g |
| Tocopheryl acetate | 2 g |

Propylene Glycol was mixed with Cremophor RH 40 and heated upto 55 to 60°C and Cyclosporin was dissolved in the resultant. Propylene glycol monolaurate was then added to the bulk mixture followed by Tocopheryl acetate and mixed. The resultant mixture was then filtered.

### Example 14

| | |
|---|---|
| Cyclosporin | 10 g |
| Propylene Glycol | 9 g |
| Cremophor RH 40 | 24 g |
| Propylene Glycol laurate | 55 g |
| Tocopheryl acetate | 2 g |

Propylene Glycol was mixed with Cremophor RH 40 and heated upto 55 to 60°C and Cyclosporin was dissolved in the resultant. Propylene glycol laurate was then added to the bulk mixture followed by Tocopheryl acetate and mixed. The resultant mixture was then filtered.

### Example 15

| | |
|---|---|
| Cyclosporin | 10 g |
| Propylene Glycol | 35 g |
| Cremophor RH 40 | 14 g |
| Propylene Glycol laurate | 35.5 g |
| Tocopheryl acetate | 0.5 g |
| Oleic acid | 5 g |

Propylene Glycol was mixed with Cremophor RH 40 and heated upto 55 to 60°C and Cyclosporin was dissolved in the resultant. Tocopheryl acetate was mixed with Oleic acid and added to Propylene glycol laurate with continuous stirring. This solution was then added to the drug solution and mixed. The resultant mixture was then filtered.

It is observed that all products obtained in the examples 6 to 10, and 12 to 15, provided upon dilution in a ratio from 1:250 to 1:1000, an average globule size in the specified range of from 200 to 600 nm, the preferred dilution medium being water.

With respect to the mixing step upon dilution it is further observed that any suitable method of mixing may be employed, an example being vortexing; nevertheless mixing simulating in vivo conditions is most preferred.

## Claims

1. A homogeneous, alcohol free composition of cyclosporin which comprises cyclosporin A in a hydrophilic carrier medium comprising propylene glycol, esters of propylene glycol with C4 to C12 fatty acids and polyoxyethylene hydrogenated castor oils wherein the ingredients are present in the following range.
| | |
|---|---|
| Cyclosporing A | 1 - 25 % w/w |
| Propylene glycol | 2 - 70 % w/w |
| Esters of propylene with C4 to C12 fatty acids | 15 - 60 % w/w |
| Polyoxyethylene hydrogenated castor oils | 5 - 20 % w/w |
which composition, upon dilution with water, yields a stable, oil-in-water emulsion, whereof the oil phase consists of cyclosporin containing globules.

2. A composition as claimed in claim 1 wherein the ingredients are present in the following range:
| | |
|---|---|
| Cyclosporing A | 5 - 15 % w/w |
| Propylene glycol | 5 - 50 % w/w |
| Esters of propylene with C4 to C12 fatty acids | 25 - 45 % w/w |
| Polyoxyethylene hydrogenated castor oils | 10 - 20 % w/w. |

3. A composition according to claim 1 or 2, wherein said globules have an average size of from 200 to 600 nm.

4. A composition according to claims 1 to 3, wherein said globules have an average size of from 200 to 300 nm.

5. A composition as claimed in any of the preceding claims, which comprises esters of propylene glycol with C12 fatty acids.

6. A composition as claimed in any of the preceding claims, which further comprises glycerol triacetate or triacetin.

7. A composition as claimed in claim 6, wherein glycerol triacetate is present in the range 0 to 10 % w/w.

8. A composition as claimed in any of the preceding claims, further comprising oleic acid.

9. A composition as claimed in claim 8, wherein oleic acid is present in the range 0 to 60 % w/w.

10. A composition as claimed in any of the preceding claims, wherein the esters of propylene glycol with C4 to C12 fatty acids are partially or completely replaced with oleic acid.

11. A composition as claimed in any of the preceding claims, further comprising antioxidants.

12. A composition as claimed in claim 11, wherein the antioxidants are present in the range 0 to 5 %.

13. A composition as claimed in claim 11 or 12, wherein said antioxidants are chosen from the group comprising butylated hydroxy anisole, butylated hydroxy toluene, tocopherylacetate, d-α-tocopheryl polyethylene glycol 1000 succinate or a mixture thereof.

14. A composition according to any of the preceding claims, wherein said composition is diluted with water in a ratio from 1:250 to 1:1000.

15. A composition as claimed in any of the preceding claims, which can be formulated as a drink solution.

16. A composition as claimed in any of the preceding claims, which is free flowing at a temperature of 15 to 45°C.

17. Use of a composition according to any of the preceding claims for the manufacture of a medicament for treating a cyclosporin indicated condition or symptom.

18. Use according to claim 17, wherein the cyclosporin indicated condition or symptom is T cell mediated immune process, allograft rejection, inflammation, or autoimmune conditions.

19. A composition claimed in any of the claims 1 to 16, which is used as a drink solution or incorporated into soft gelatin capsules or hard gelatin capsules or hard cellulose capsules or packed in a suitable device to affect measurable unit dosage dispending.

## Patentansprüche

1. Homogene, alkoholfreie Zusammensetzung von Cyclosporin, die Cyclosporin A in einem hydrophilen Trägermedium umfaßt, umfassend Propylenglykol, Ester von Propylenglykol mit C4- bis C12-Fettsäuren und Polyoxyethylen-hydrierten Rizinusöle, wobei die Inhaltsstoffe im folgenden Bereich vorliegen:
| | |
|---|---|
| Cyclosporin A | 1 - 25 % Gew./Gew. |
| Propylenglykol | 2 - 70 % Gew./Gew. |
| Ester von Propylenglykol mit C4- bis C12-Fettsäuren | 15 - 60 % Gew./Gew. |
| Polyoxyethylen-hydrierte Rizinusöle | 5 - 20 % GewJGew., |
wobei die Zusammensetzung bei Verdünnung mit Wasser eine stabile Öl-in-Wasser-Emulsion ergibt, wovon die Ölphase aus Cyclosporin-enthaltenden Kügelchen besteht.

2. Zusammensetzung nach Anspruch 1, wobei die Inhaltsstoffe im folgenden Bereich vorliegen:
| | |
|---|---|
| Cyclosporin A | 5 - 15 % Gew./Gew. |
| Propylenglykol | 5-50 % Gew./Gew. |
| Ester von Propylenglykol mit C4- bis C12-Fettsäuren | 25 - 45 % Gew./Gew. |
| Polyoxyethylen-hydrierte Rizinusöle | 10 - 20 % Gew./Gew. |

3. Zusammensetzung nach Anspruch 1 oder 2, wobei besagte Kügelchen eine durchschnittliche Größe von 200 bis 600 nm haben.

4. Zusammensetzung nach Anspruch 1 bis 3, wobei besagte Kügelchen eine durchschnittliche Größe von 200 bis 300 nm haben.

5. Zusammensetzung nach einem der vorangehenden Ansprüche, die Ester von Propylenglykol mit C12-Fettsäuren umfaßt.

6. Zusammensetzung nach einem der vorangehenden Ansprüche, die weiterhin Glyceroltriacetat oder Triacetin umfaßt.

7. Zusammensetzung nach Anspruch 6, wobei Glyceroltriacetat im Bereich von 0 bis 10% Gew./Gew. vorliegt.

8. Zusammensetzung nach einem der vorangehenden Ansprüche, weiterhin umfassend Ölsäure.

9. Zusammensetzung nach Anspruch 8, wobei die Ölsäure im Bereich von 0 bis 60% Gew./Gew. vorliegt.

10. Zusammensetzung nach einem der vorangehenden Ansprüche, wobei die Ester von Propylenglykol mit C4- bis C12-Fettsäuren teilweise oder vollständig durch Ölsäure ersetzt sind.

11. Zusammensetzung nach einem der vorangehenden Ansprüche, weiterhin umfassend Antioxidantien.

12. Zusammensetzung nach Anspruch 11, wobei die Antioxidantien im Bereich von 0 bis 5% vorliegen.

13. Zusammensetzung nach Anspruch 11 oder 12, wobei besagte Antioxidantien ausgewählt sind aus der Gruppe, umfassend butyliertes Hydroxyanisol, butyliertes Hydroxytoluol, Tocopherylacetat, d-α-Tocopheryl-Polyethylenglycol-1000-Succinat oder eine Mischung davon.

14. Zusammensetzung nach einem der vorangehenden Ansprüche, wobei besagte Zusammensetzung mit Wasser in einem Verhältnis von 1:250 bis 1:1000 verdünnt ist.

15. Zusammensetzung nach einem der vorangehenden Ansprüche, die als eine Trinklösung formuliert werden kann.

16. Zusammensetzung nach einem der vorangehenden Ansprüche, die bei einer Temperatur von 15 bis 45°C frei fließend ist.

17. Verwendung einer Zusammensetzung nach einem der vorangehenden Ansprüche zur Herstellung eines Medikaments zum Behandeln eines Cyclosporin-indizierten Zustands oder Symptoms.

18. Verwendung nach Anspruch 17, wobei der Cyclosporin-indizierte Zustand oder Symptom ein T-Zell-vermittelter Immunprozeß, eine Allogran-Abstoßung, Entzündung oder Autoimmunzustände ist.

19. Zusammensetzung nach einem der Ansprüche 1 bis 16, die als eine Trinklösung verwendet wird oder in weiche Gelatinekapseln oder harte Gelatinekapseln oder harte Cellulosekapseln eingebaut ist oder in einer geeigneten Vorrichtung verpackt ist, um eine meßbare Einheitsdosierungsabgabe zu bewirken.

## Revendications

1. Composition homogène, exempte d'alcool, de cyclosporine, qui comprend la cyclosporine A dans un milieu support hydrophile, comprenant du propylèneglycol, des esters de propylèneglycol avec des acides gras en C4 à C12 et des huiles de ricin hydrogénées-polyoxyéthylène, où les ingrédients sont présents dans l'intervalle suivant :
| | |
|---|---|
| Cyclosporine | 1 - 25% p/p |
| Propylèneglycol | 2 - 70% p/p |
| Esters de propylèneglycol avec des acides gras en C4 à C12 | 15 - 60% p/p |
| Huiles de ricin hydrogénées polyoxyéthylène | 5 à 20% p/p |
laquelle composition, par dilution avec de l'eau, conduit à une émulsion huile-dans-eau stable, où la phase huileuse consiste en des globules contenant la cyclosporine.

2. Composition selon la revendication 1, dans laquelle les ingrédients sont présents dans l'intervalle suivant :
| | |
|---|---|
| Cyclosporine | 5 - 15% p/p |
| Propylèneglycol | 5 - 50% p/p |
| Esters de propylèneglycol avec des acides gras en C4 à C12 | 25 - 45% p/p |
| Huiles de ricin hydrogénées polyoxyéthylène | 10 à 20% p/p |

3. Composition selon la revendication 1 ou 2, dans laquelle lesdits globules ont une taille moyenne allant de 200 à 600 nm.

4. Composition selon la revendication 1 à 3, dans laquelle lesdits globules ont une taille moyenne allant de 200 à 300 nm.

5. Composition selon l'une quelconque des revendications précédentes, qui comprend des esters de propylèneglycol avec des acides gras en C12.

6. Composition selon l'une quelconque des revendications précédentes, qui comprend en outre, le triacétate de glycérol ou triacétine.

7. Composition selon la revendication 6, dans laquelle le triacétate de glycérol est présent dans l'intervalle allant de 0 à 10% p/p.

8. Composition selon l'une quelconque des revendications précédentes, comprenant en outre l'acide oléique.

9. Composition selon la revendication 8, dans laquelle l'acide oléique est présent dans l'intervalle allant de 0 à 60% p/p.

10. Composition selon l'une quelconque des revendications précédentes, dans laquelle les esters du propylèneglycol avec des acides gras en C4 à C12 sont pratiquement ou complètement remplacés par l'acide oléique.

11. Composition selon l'une quelconque des revendications précédentes, comprenant en outre des antioxydants.

12. Composition selon la revendication 11, dans laquelle les antioxydants sont présents dans l'intervalle allant de 0 à 5%.

13. Composition selon la revendication 11 ou 12, dans laquelle lesdits antioxydants sont choisis parmi le groupe comprenant l'hydroxyanisole butylé, l'hydroxytoluène butylé, l'acétate de tocophéryle, le succinate de d-α-tocophéryle-polyéthylèneglycol 1000 ou un mélange de ceux-ci.

14. Composition selon l'une quelconque des revendications précédentes, dans laquelle ladite composition est diluée avec de l'eau en un rapport allant de 1 : 250 à 1 : 1000.

15. Composition selon l'une quelconque des revendications précédentes, qui peut être formulée sous la forme d'une solution buvable.

16. Composition selon l'une quelconque des revendications précédentes, qui est fluide à une température allant de 15 à 45°C.

17. Utilisation d'une composition selon l'une quelconque des revendications précédentes, pour la préparation d'un médicament pour traiter un état ou des symptômes, pour lesquels la cyclosporine est indiquée.

18. Utilisation selon la revendication 17, dans laquelle l'état ou les symptômes, pour lesquels la cyclosporine est indiquée, est un processus immun à médiation par cellule T, un rejet d'allogreffe, une inflammation ou des états autoimmuns.

19. Composition selon l'une quelconque des revendications 1 à 16, qui est utilisée sous la forme d'une solution buvable ou qui est incorporée dans des capsules de gélatine molle ou des capsules de gélatine dure ou des capsules de cellulose dure ou emballée dans un dispositif approprié pour réaliser une administration sous forme d'unité de dosage mesurable.
